(19)

Europäisches Patentamt
European Patent Office
Office européen des brevets

(11) **EP 3 838 144 A1**

(12) **EUROPEAN PATENT APPLICATION**

(43) Date of publication:
**23.06.2021 Bulletin 2021/25**

(51) Int Cl.:
***A61B 5/1455*** (2006.01)   ***A61B 5/00*** (2006.01)

(21) Application number: **19217847.3**

(22) Date of filing: **19.12.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(71) Applicant: **Koninklijke Philips N.V.**
**5656 AG Eindhoven (NL)**

(72) Inventors:
• **VERKRUIJSSE, Willem**
**5656 AE Eindhoven (NL)**

• **WANG, Wenjing**
**5656 AE Eindhoven (NL)**
• **MOCO, Andreia**
**5656 AE Eindhoven (NL)**
• **LAMBERT, Niek**
**5656 AE Eindhoven (NL)**
• **MENA BENITO, Estrella**
**5656 AE Eindhoven (NL)**

(74) Representative: **Philips Intellectual Property & Standards**
**High Tech Campus 5**
**5656 AE Eindhoven (NL)**

(54) **DEVICE, SYSTEM AND METHOD FOR DETERMINING OXYGEN SATURATION OF A SUBJECT**

(57)    The present invention relates to a device (100), system (500) and method for determining SpO2 (160) of a subject. Two types of PPG measurements, widefield PPG with a homogenous illumination and/or a spot pattern and radial PPG with a spot illumination, are used to quantify the discrepancy in penetration depths for electromagnetic radiation in the red spectral range and infrared spectral range. This discrepancy in penetration depth is then used to find a more stable ratio of ratios, RR, and thus a more accurate SpO2 (160).

FIG.11

EP 3 838 144 A1

## Description

FIELD OF THE INVENTION

The present invention relates to a device, system and method for determining oxygen saturation of a subject.

BACKGROUND OF THE INVENTION

Pulse oximeters measure arterial oxygen saturation (SpO2) continuously in a non-invasive way and are nowadays routinely used in many clinical practices. Further, pulse oximetry has become widely available in various aspects of health care in general, including neonatal care where artificial oxygen supply is common.

The accuracy of pulse oximetry is typically insufficient for premature infants (often neonatal intensive care unit (NICU) patients) in which the clinically safest saturation level is believed to be around 95% instead of 100% in adults. A very fine balance is required in neonates between supplying too much oxygen with a risk of retinopathy of prematurity (ROP) and too little oxygen which can cause brain damage or death as investigated, e.g., in A. Hellstrom et al: Retinopathy of prematurity, The Lancet 382 (9902), 2013 and O.D. Saugstad and D. Aune: Optimal Oxygenation of Extremely Low Birth Weight Infants: A Meta-Analysis and Systematic Review of the Oxygen Saturation Target Studies, Neonatology 105,2014.

Partly due to the relative inaccuracy of pulse oximeters the actual target saturation levels are not precisely known and very large international studies are undertaken to determine this. In general, there is an urgent need for pulse oximeters with higher accuracies.

A fundamental problem in state of the art pulse oximetry is that it is tacitly assumed that the used wavelengths 'see' the same pulsatile arteriolar vessels and that the relative photoplethysmography (PPG) amplitudes reflect the saturation of the blood therein. If red and near-infrared light have different penetration depths (e.g., due to absorption by non-pulsatile venous blood) a resulting difference in relative PPG amplitude is falsely interpreted as caused by a different SpO2 level.

SUMMARY OF THE INVENTION

It is an object of the present invention to provide a more accurate device, method and system for determining SpO2 of a subject.

In a first aspect of the present invention a device for determining SpO2 of a subject is presented that comprises a processing unit configured to:

- obtain a first and second detection signal derived from detected electromagnetic radiation at different wavelengths transmitted through or reflected from a skin region of the subject illuminated by spot illumination;

- obtain a third and fourth detection signal derived from detected electromagnetic radiation at said different wavelengths transmitted through or reflected from said skin region of the subject illuminated by homogenous illumination and/or by a spot pattern of illumination, wherein the third detection signal is derived from detected electromagnetic radiation at the same wavelength as the first detection signal and the fourth detection signal is derived from detected electromagnetic radiation at the same wavelength as the second detection signal;

- determine a first ratio of ratios (RR1) from the first and second detection signal and a second ratio of ratios (RR2) from the third and fourth detection signal;

- determine a first normalized signal by calculating the ratio of the first detection signal to the third detection signal and a second normalized signal by calculating the ratio of the second detection signal to the fourth detection signal;

- determine a penetration depth ratio (PDR) by calculating the ratio of the first normalized signal to the second normalized signal;

- correct the RR1 and the RR2 using the PDR to compensate for the discrepancy in penetration depth between said different wavelengths; and

- determine the SpO2 from the corrected RR1 and/or the corrected RR2.

According to another aspect of the present invention, a system for determining SpO2 of a subject is presented that comprises, besides the above described device, an illumination unit configured to emit a narrow beam of electromagnetic radiation to illuminate the skin region of the subject by a spot illumination, an optical diffuser that can selectively be arranged within or outside of the path of the emitted light of the illumination unit, wherein the optical diffuser is configured to diffuse the electromagnetic radiation emitted by the illumination unit to illuminate the skin region of the subject homogenously and/or by a spot pattern and a detection unit configured to detect the electromagnetic radiation transmitted through or reflected from the skin region of the subject and to derive detection signals from the detected electromagnetic radiation.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed device, in particular as defined in the dependent claims and as disclosed herein.

The present invention is based on the idea to combine two types of PPG measurements (widefield PPG and radial PPG) to quantify the discrepancy in penetration depths for electromagnetic radiation in different spectral ranges. This discrepancy can then be used to find a more stable RR, and thus a more accurate SpO2 level largely independent on the above-mentioned inaccuracies. For the determination of SpO2 the standard model of using the linear relation between SpO2 and RR is used. A more detailed explanation will be given below in the description of the drawings.

Widefield PPG means in the discussed context the measurement type where the skin of the subject is illuminated homogenously and/or by structured light (a spot pattern of dots, circles, stripes, etc.), while radial PPG means in said context the measurement type where the skin of the subject is illuminated by spot illumination (such as by a laser).

By combining radial PPG amplitudes with widefield PPG amplitudes for two different wavelengths, a relative penetration index (called PDR) can be obtained and then be used to compute a SpO2 level with much higher accuracy compared to standard pulse oximeters.

This also provides a novel way of providing a depth measure for the PPG source which is not dependent on individual differences caused by cardiac output or arterial stiffness. This measure can be of importance to assess centralization, vasodilation or constriction, in wound healing, or pre and post vascular surgeries.

As defined above, the RR1 is determined from the first and second detection signal. Hence, this RR1 may also be referred to as radial RR as the first and second detection signal are both derived from a radial PPG measurement type. The RR2 is determined from the third and fourth detection signal. Hence, the RR2 may also be referred to as widefield RR as the third and fourth detection signal are both derived from a widefield PPG measurement type.

Further, the first normalized signal is determined by calculating the ratio of the first detection signal to the third detection signal. These two detection signals are derived from electromagnetic radiation with the same wavelength, such as exemplarily electromagnetic radiation in the near-infrared spectral range. Hence, this first normalized signal may indicate a normalized signal relating to electromagnetic radiation in the near-infrared spectral range. In said context, the first normalized signal is a measure for the penetration depth of electromagnetic radiation in the near-infrared spectral range in the skin of the subject.

The second normalized signal is determined by calculating the ratio of the second detection signal to the fourth detection signal. These two detection signals are also derived from electromagnetic radiation with the same wavelength, but this wavelength has to be different from the underlying used wavelength of the first and the third detection signal. Thus, the second and fourth detection signal may be exemplarily derived from electromagnetic radiation in the red spectral range. Hence, the second normalized signal may indicate a normalized signal relating to electromagnetic radiation in the red spectral range. In said context, the second normalized signal is a measure for the penetration depth of electromagnetic radiation in the red spectral range in the skin of the subject.

The PDR is determined by calculating the ratio of the first normalized signal to the second normalized signal and is thus a measure for the relative penetration depths.

According to another embodiment, the processing unit is configured to derive, in case of a spot pattern of illumination, the third and fourth detection signals by a spatial integral of all the electromagnetic radiation transmitted through or reflected from said skin region.

Widefield PPG is typically measured by using a light source that emits electromagnetic radiation homogenously onto a skin region of a subject. However, using structured light, i.e., a pattern of dots, stripes, circles, etc. works as well. In fact, any non-homogenous illumination pattern can work, albeit with great deconvolution work.

Further, even using just a spot illumination (i.e., one laser spot on the skin of the subject) and determining the spatial integral of all light reflected back from the skin of the subject is also suitable for providing widefield PPG.

In comparison to using homogenous illumination, using a structured pattern or just one illumination spot provides the advantage to gain more signal strength in a few pixels, which may be suitable to obtain more accurate PPG signals.

Hence, it may also be a viable option to use a combination of a homogenous illumination and spot illumination or illumination by structured light. This may be interesting if the processing unit is further used for PPG imaging as the homogenous illumination in turn provides a better spatial resolution compared to a spot illumination or an illumination by structured light.

According to one embodiment, the processing unit is configured to correct the RR1 and/or the RR2 by use of reference ratio of ratios ($RR_{ref}$) and reference penetration depth ratios ($PDR_{ref}$).

These reference ratios may be determined by models, numerical simulations, but also by empirical measurements on a large number of individuals. Preferably, the determined RR1 or RR2 and the corresponding determined PDR are both compared to $RR_{ref}$ and $PDR_{ref}$.

Further, it shall be understood that the $RR_{ref}$ are preferably split into reference ratio of ratios for the RR1 and for the RR2, respectively, as the ratio of ratios determined from radial PPG and the ratio of ratios determined from widefield PPG are typically different.

According to another embodiment, the processing unit is configured to correct the RR1 and/or the RR2 value by comparing said RR1 and/or said RR2 and said PDR to a lookup table of reference ratio of ratios $RR_{ref}$ and reference penetration depth ratios $PDR_{ref}$. Preferably, said lookup table is split into a lookup table for the reference ratios for RR1 (radial RR) and a lookup table for reference ratios for RR2 (widefield RR).

According to another embodiment, the processing unit is configured to use calibration curves describing the relationship between reference ratio of ratios $RR_{ref}$ and reference penetration depth ratio $PDR_{ref}$ for different SpO2 values to compare the PDR value and the RR1 value and/or RR2 value to said calibration curves.

These calibration curves are preferably determined from the $RR_{ref}$ and from the $PDR_{ref}$ stored in the respective lookup tables for RR1 (radial RR) and RR2 (widefield RR). Thus, these lookup tables may comprise three columns, wherein the first column comprises $RR_{ref}$, the second column $PDR_{ref}$ and the third column the correct SpO2 values. Based on these lookup tables, the calibration curves may be determined manually by a user or automatically by the processing unit. The calibration curves are then preferably visualized in a diagram with an axis of ordinate and an axis of abscissae, wherein the axis of ordinate illustrates the $RR_{ref}$, and the axis of abscissae illustrates the $PDR_{ref}$. Various calibration curves for different SpO2 values may then be visualized in one diagram. A more detailed explanation is given later with reference to the description of the figures.

According to another embodiment, the processing unit is configured to select a matching calibration curve to correct the RR1 and/or the RR2 by extrapolating said matching curve to the $PDR_{ref}$ equal to 1 and setting the RR1 and/or the RR2 to the corresponding $RR_{ref}$.

The determined RR1 or the determined RR2 and the corresponding PDR is thus compared to the plurality of calibration curves and a matching calibration curve is selected by the processing unit. This may be just done by selecting the matching curve, which is closest to the data point obtained in the above-mentioned diagram when the determined RR1 or the determined RR2 and the corresponding PDR are visualized as one data point in said diagram. By selecting said matching curve and extrapolating said curve to the $PDR_{ref}$ equal to 1 and setting the RR1 or the RR2 to the corresponding $RR_{ref}$ as the corrected RR1 or the corrected RR2, the ratio of ratios are corrected for the unequal penetration depths of electromagnetic radiation in the red and infrared spectral range.

The claimed system is not limited to the use of one illumination unit or one detection unit. Nevertheless, at least one illumination unit has to be a point illumination source (such as a laser) to provide the possibility to perform radial PPG measurements.

The above-mentioned embodiment of the system provides the advantage that only one illumination unit is needed. As the optical diffuser can be selectively arranged within or outside of the path of the emitted light of the illumination unit, either a widefield PPG or a radial PPG measurement can be performed. Thus, a compact system is provided for accurate SpO2 measurements. The narrow beam of electromagnetic radiation allows obtaining a discrete spot on the skin of the subject, wherein the spot may be a dot, a circle, a line, etc.

Preferably, the illumination unit is further configured to emit electromagnetic radiation at at least two different wavelengths and/or to alternately emit red light and infrared light. Thus, a first measurement may be performed by measuring radial PPG with red light and a second measurement by measuring radial PPG with infrared light. Then, the optical diffuser may be arranged inside of the path of the emitted light to diffuse the light to obtain a structured pattern of illumination and/or a homogenous illumination on the skin of the subject. Then, a third measurement may be performed by measuring widefield PPG with red light and a fourth measurement by measuring widefield PPG with infrared light. It should be noted that rather than red and infrared light one may also choose two or more infrared wavelengths in applications when visible light is not desired (e.g., for sleep monitoring, where visible light may disturb the sleeping person). Nevertheless, red light is often preferred since at this wavelength the contrast for SpO2 is larger than for combinations of infrared wavelengths.

According to another embodiment, the illumination unit and the detection unit are either both in direct physical contact to the skin of the subject or not in direct physical contact to the skin of the subject.

Hence, it shall be understood that the system is not limited to be used as a remote PPG setup as it can also be used as contact PPG, where the illumination unit and the detection unit are directly attached to the skin of the subject (e.g., as a finger clip).

Furthermore, it should also be understood that the widefield PPG signals may be replaced by signals with a very small radial (source-detector) distance as this signal is very similar in the sense that it is predominantly probing the upper skin layers (as the widefield PPG signals).

According to a further aspect, the above-mentioned system may be modified such that the system not only comprises a first illumination unit configured to emit a narrow beam of electromagnetic radiation to illuminate the skin region of the subject by a spot illumination, but also a second illumination unit configured to emit a homogenous illumination profile of electromagnetic radiation and/or structured light to illuminate the skin region of the subject homogenously and/or by structured light. Structured light means in said context a pattern of electromagnetic radiation, i.e., a pattern of dots, circles, stripes, etc.

According to this aspect, no optical diffuser is needed as the radial PPG measurement may be performed by the use of the first illumination unit and the wide field PPG may be performed by use of the second illumination unit.

It shall be understood that said modified system may have the same embodiments that have been discussed with reference to the system with only one illumination unit.

According to another embodiment, the detection unit is an optical sensor and comprises a plurality of detection elements, in particular an array of photo diodes, a CCD array or a CMOS array. If the detection unit is in contact with the skin of the subject as a contact-device, a widefield PPG measurement requires further that an array of detection units is used, rather than just one.

Further advantages result from the description and the attached drawings. It shall be understood that the features mentioned above and below may be used not only in the combinations indicated, but also in other combinations or as a whole, without leaving the framework of this invention.

BRIEF DESCRIPTION OF THE DRAWINGS

These and other aspects of the invention will be apparent from and elucidated with reference to the embodiment(s) described hereinafter. In the following drawings

Fig. 1 shows a diagram illustrating the blood absorption coefficient of a subject in dependence on the wavelength of electromagnetic radiation transmitted through or reflected from skin of the subject;

Fig. 2 shows a diagram illustrating a reference SpO2 value of a subject in dependence on the RR;

Figs. 3A and 3B show schematic diagrams illustrating two tacit assumptions in pulse oximetry;

Figs. 4A and 4B show schematic diagrams illustrating the effect of an increase in venous blood on the penetration depths of red and infrared electromagnetic radiation;

Figs. 5A and 5B show schematic diagrams illustrating the effect of an equal penetration depth of red and infrared electromagnetic radiation on a SpO2 measurement;

Figs. 6A and 6B show schematic diagrams illustrating the effect of an unequal penetration depth of red and infrared radiation on a SpO2 measurement;

Figs. 7A and 7B show schematic diagrams illustrating the path of electromagnetic radiation through skin of a subject for widefield PPG and radial PPG;

Figs. 8A and 8B show schematic diagrams illustrating the detected reflected light of widefield PPG and radial PPG;

Fig. 9 shows a schematic diagram of Monte Carlo simulations of the paths of photons through skin of a subject for radial PPG;

Figs. 10A and 10B show schematic diagrams of a system for determining a SpO2 of a subject according to the present invention;

Fig. 11 shows a flowchart illustrating a method to be executed by the device for determining a SpO2 of a subject according to the present invention;

Fig. 12 shows schematic diagrams illustrating the first processing step of the detection signals obtained by the device;

Figs. 13A and 13B show schematic diagrams illustrating the influence of different skin layers on the measured signals;

Fig. 14 shows a diagram illustrating the PDR in dependence on the radial distance;

Fig. 15 shows a diagram illustrating the RR1 in dependence on the PDR;

Fig. 16 shows a diagram illustrating the RR2 in dependence on the PDR;

Fig. 17 shows a diagram illustrating the RR1 in dependence on the PDR for various parameters;

Fig. 18 shows a diagram illustrating the RR2 in dependence on the PDR for various parameters;

Fig. 19 shows a diagram (Fig. 19A) and a lookup table (Fig. 19B) illustrating an example of correction of RR1;

Fig. 20 shows a diagram showing a constant corrected RR1;

Fig. 21 shows a diagram illustrating an example of correction of RR2;

Fig. 22 shows a diagram showing a constant corrected RR2; and

Figs. 23A-23D show diagrams illustrating the determined SpO2 versus RR.

DETAILED DESCRIPTION OF EMBODIMENTS

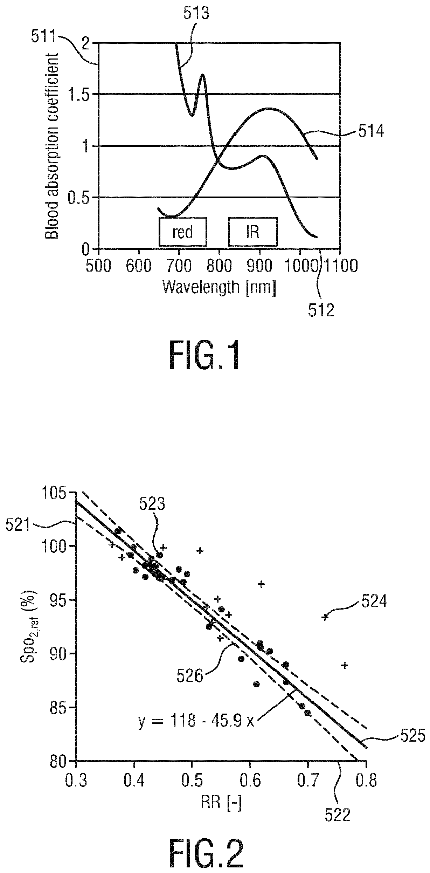

Fig. 1 shows a diagram illustrating the blood absorption coefficient of a subject in dependence on the wavelength of electromagnetic radiation transmitted through or reflected from skin of the subject. For this purpose, the axis of ordinate 511 illustrates the blood absorption coefficient and the axis of abscissae 512 illustrates the wavelength of said electromagnetic radiation in the spectral range from 500 nm to 1100 nm (visible to near-infrared spectral range). A first curve 513 illustrates the absorption of hemoglobin (Hb) and a second curve 514 illustrates the absorption of oxygenated (HbO2). Both constituents are part of the blood contained in the vessels of the skin.

Pulse oximetry is based on the straightforward principle that HbO2 and Hb absorb electromagnetic radiation in the red and infrared spectral range differently as shown by the different curves 513, 514 in Fig. 1. The ability of pulse oximetry to detect SpO2 of only arterial blood is further based on the principle that the amount of red ($\lambda1$) and infrared light ($\lambda2$) absorbed fluctuates with the cardiac cycle as the arterial blood volume increases during systole and decreases during diastole. From the resulting modulated light intensities (which is known as PPG) a ratio of ratios (RR) is calculated by the following formula

$$RR - \frac{RR_{\lambda1}}{RR_{\lambda2}} - \frac{AC_{\lambda1}/DC_{\lambda1}}{AC_{\lambda2}/DC_{\lambda2}}, \quad (1)$$

where the ratio of pulsatile signals (AC) and non-pulsatile signals (DC) of one specific wavelength $\lambda1$ is normalized to the ratio of pulsatile and non-pulsatile signals of the other wavelength $\lambda2$.

This RR can be regarded as being nearly linear with respect to SpO2 of the subj ect

$$SpO_2 = C_1 - C_2 RR, \quad (2)$$

where C1 and C2 are linear equation coefficients. Thus, SpO2 can be obtained by measuring RR. This is a standard model of determining the SpO2 value of a subject and also used here. The linear relationship (2) is the simplest form of describing the relationship between SpO2 and RR. Different relationships, such as, e.g., 2nd or more degree polynomials may also be used, or even lookup-tables.

Fig. 2 shows a diagram illustrating a reference SpO2 ($SpO_{2,ref}$) value of a subject in dependence on the RR value. For this purpose, the axis of ordinate 521 illustrates the SpO2 value in percent (%) and the axis of abscissae 522 illustrates the RR. The linear dependency of the SpO2 value on the RR value is clearly visible. Only filtered measured data 523 represented by circles are used for the linear regression 525. The dashed line 526 represents a corresponding 99 % confidence interval and the measured discarded data points 524 not used in the calibration are also shown.

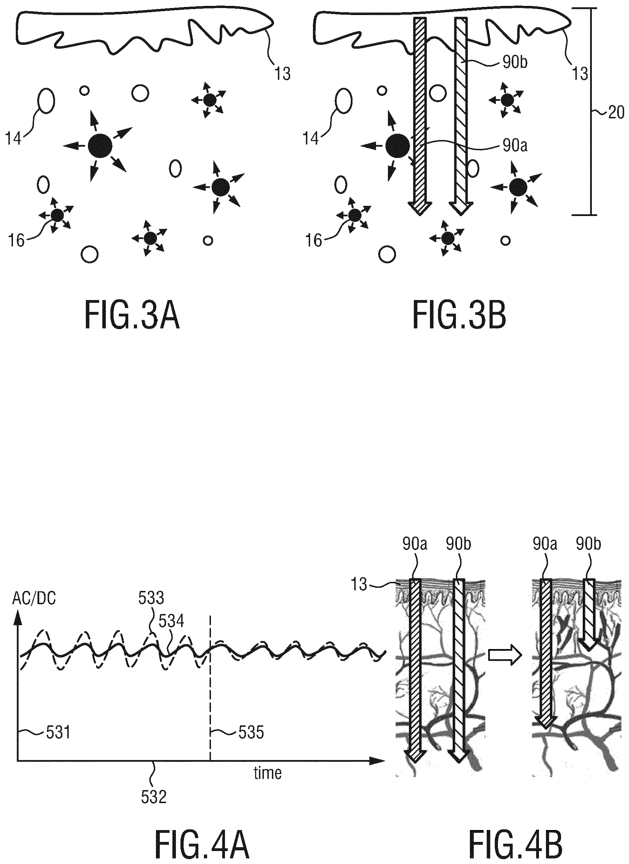

Figs. 3A and 3B show schematic diagrams illustrating two tacit assumptions in pulse oximetry. The first assumption is that only arterial blood volume is pulsatile. The epidermis 13 of the skin of a subject and the underlying venules 14 and arterioles 16 are illustrated in Fig. 3A. The pulsatile component of the arterioles 16 is indicated by the arrows around the arterioles 16 pointing away from the respective arterioles.

The second assumption constantly made in pulse oximetry is that the used wavelengths $\lambda1$ and $\lambda2$ 'see' the same vasculature. This is illustrated by the large arrows in Fig. 3B indicating the electromagnetic radiation 90a, 90b in the red ($\lambda1$) and near-infrared ($\lambda2$) spectral range, wherein the lengths of said arrows indicate the respective penetration depths 20. Especially this second assumption is a constant problem arising in classical pulse oximetry and addressed by the present invention and further explained with reference to the subsequent figures.

Figs. 4A and 4B show schematic diagrams illustrating the effect of an increase in venous blood on the penetration depths of red and infrared electromagnetic radiation. The diagram shown in Fig. 4A shows on the axis of ordinate 531 the ratio of the pulsatile to non-pulsatile components (AC/DC) of a PPG signal and on the axis of abscissae 532 the time. The first curve 533 illustrates the ratio AC/DC (or the relative amplitude of the PPG signal) for near-infrared radiation, while the second curve 534 illustrates the ratio AC/DC (or the relative amplitude of the PPG signal) for electromagnetic radiation in the red spectral range.

At a certain time point illustrated by the vertical dashed line 535 in Fig. 4A, an increase in venous blood (which is static, not pulsatile) affects the penetration depths of both red and infrared radiation, but not to the same extent. This can be seen by comparing the lengths of the arrows in Fig. 4B illustrating the penetration depths 20 of electromagnetic radiation 90a in the red spectral range and electromagnetic radiation 90b in the infrared spectral range before (left side of Fig. 4B) and after (right side of Fig. 4B) the increase of venous blood. It can be seen that the infrared light does not reach the deeper pulsatile vessels anymore which results in a lower relative PPG amplitude as shown in Fig. 4A for times larger than the time point illustrated by the vertical dashed line 535. On the contrary, the red PPG amplitude is affected much less as can be seen by comparing the lengths of the corresponding arrows on the left side and right side of Fig. 4B.

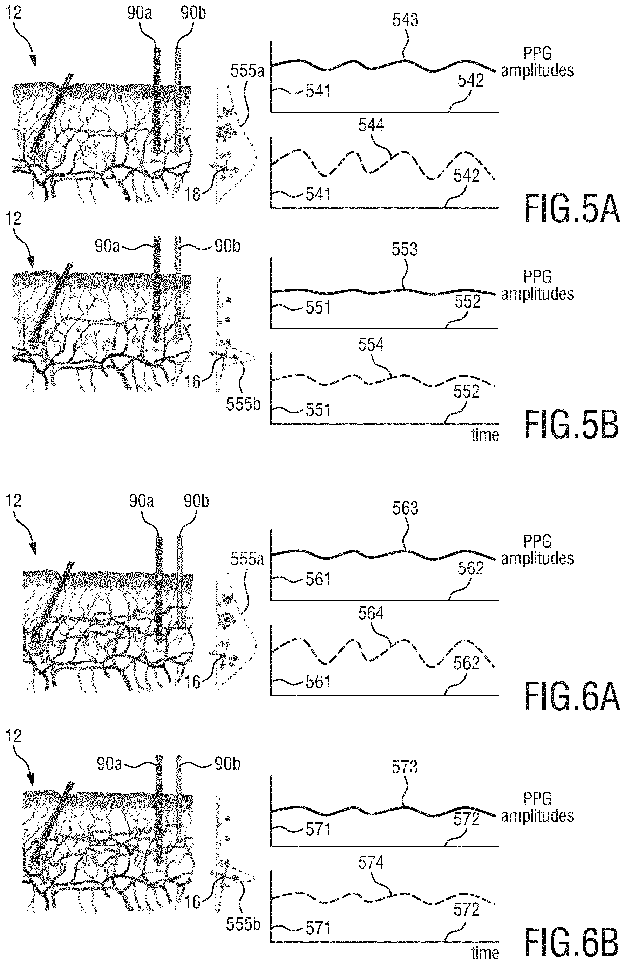

If the pulsatile strength of vessels is homogenous throughout the skin depth, the problem might be small, even if the wavelengths have different penetration depths. However, when pulsatile vessels are in a distinct layer, it can cause a problem. This is illustrated in the following Figs. 5A, 5B and 6A, 6B.

Figs. 5A and 5B show schematic diagrams illustrating the effect of an equal penetration depth of red and infrared electromagnetic radiation on a SpO2 measurement. The axes of ordinate 541, 551 illustrate the relative PPG amplitude and the axes of abscissae 542, 552 illustrate the time (on the right side of Figs. 5A and 5B, respectively). The first curve 543 of Fig. 5A and the first curve 553 of Fig. 5B illustrate the PPG amplitude derived from red electromagnetic radiation 90a transmitted through or reflected from skin region 12 of a subject, while the second curve 544 of Fig. 5A and the second curve 554 of Fig. 5B illustrate the PPG amplitude derived from near-infrared electromagnetic radiation 90b transmitted through or reflected from skin region 12 of the subject.

From both measurements shown in Figs. 5A and 5B a RR of 0.5 is calculated, which results in a SpO2 of 100 %. The difference between Figs. 5A and 5B is just that the pulsatile profile 555a shown in the middle column of Fig. 5A is much more homogenous than the pulsatile profile 555b shown in Fig. 5B, which means that the pulsatile vessels shown in the middle column of Fig. 5A are more homogenously distributed along the penetration depth of the electromagnetic radiation 90 compared to the pulsatile vessels shown in the middle column of Fig. 5B. This, however, does not influence the result of SpO2 in the case of equal penetration depths. SpO2 is accurately measured, regardless whether the pulsatile profiles 555a, 555b are homogenous or at discrete depths.

This is distinctively different in Figs. 6A and 6B, which show schematic diagrams illustrating the effect of an unequal penetration depth of red and infrared electromagnetic radiation 90a,b on a SpO2 measurement.

The axes of ordinate 561, 571 illustrate the PPG amplitude and the axes of abscissae 562, 572 illustrate again the time. The first curve 563 of Fig. 6A and the first curve 573 of Fig. 6B illustrate the PPG amplitudes of red electromagnetic radiation 90a transmitted through or reflected from skin 12 of a subject, while the second curve 564 of Fig. 6A and the second curve 574 of Fig. 6B illustrate the PPG amplitudes of near-infrared electromagnetic radiation 90b transmitted through or reflected from skin 12 of the subject.

The difference between Figs. 6A and 6B is the same that has already been discussed with reference to Figs. 5A and 5B: the pulsatile profile 555a shown in the middle column of Fig. 6A is much more homogenously distributed along the penetration depths of the electromagnetic radiation than the pulsatile profile 555b shown in Fig. 6B.

A RR of 0.6 is calculated for the exemplary scenario illustrated in Fig. 6A resulting in a SpO2 value of 90%, while a RR larger than 0.6 is calculated for the exemplary scenario illustrated in Fig. 6B resulting in a SpO2 larger than 90 %. Hence, if penetration depths are unequal, an error in SpO2 can occur. This may in particular happen when the pulsatile profiles 555a, 555b are markedly different.

The inventors realized said problem and hypothesized that the relatively poor accuracies of standard pulse oximeters are at least partly caused by the described unequal/changing penetration depths.

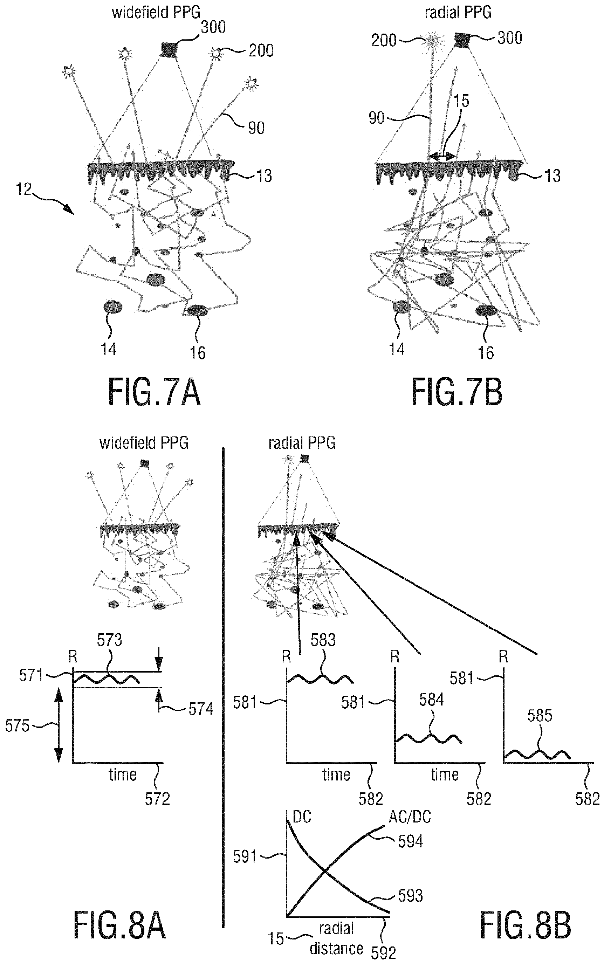

Figs. 7A and 7B show schematic diagrams illustrating the path of electromagnetic radiation 90 through skin region 12 of a subject for widefield PPG and radial PPG.

Widefield PPG is the mode that is commonly used in camera mode for various years, where the illumination by electromagnetic radiation 90 is homogenously distributed across the skin region 12 and the PPG signal is measured across that same skin area. This mode is shown in Fig. 7A. The electromagnetic radiation 90 travels through the skin region 12 with various venules 14 and arterioles 16 before getting detected by a detection unit 300, such as a camera. The detected electromagnetic radiation and the derived PPG signal is an average of all the electromagnetic radiation 90 detected by the camera 300.

Fig. 7B shows a radial PPG mode. Radial PPG is in principle quite similar to conventional contact probe PPG measurements. The skin region 12 is illuminated by a spot (such as a circle, a stripe, a dot, etc.) and the PPG signal is measured several millimeters away from that illumination spot on the skin region 12. This radial distance 15 between the illumination spot on the skin region 12 and the spot from which the PPG signal is measured is exemplarily illustrated for one reflected beam in Fig. 7B. Hence, the radPPG signal is in general a signal, which depends on the radial distance 15. In other words, the electromagnetic radiation reflected from the skin region 12 of the subject is reflected from said skin region 12 by entering into the skin region 12 through the epidermis at the illumination spot of the illumination unit 200, getting reflected from constituents of the skin region 12 and exiting the skin region 12 through the epidermis 13 at a radial distance 15 from the illumination spot. In other words, the electromagnetic radiation detected by the detection unit 300 is scattered back from the skin region 12 and collects pulsatile information from the different venules 14 and arterioles 16 located in the skin region 12 below the epidermis 13.

It will be shown below that the present invention combines the results obtained from these two different measurement modes (radial PPG and widefield PPG).

Figs. 8A and 8B show schematic diagrams illustrating the detected reflected light of widefield PPG and radial PPG. The schematic diagrams on the top of Figs. 8A and 8B are the same that have already been discussed with reference to previous Figs. 7A and 7B.

Fig. 8A shows (on the bottom) a diagram illustrating the detected reflected light of a widefield PPG setup. For this purpose, the axis of ordinate 571 illustrates the reflected detected light and the axis of abscissae 572 illustrates the measurement time. The curve 573 illustrates the reflected detected light, which comprises a DC component 575 and an AC component 574. The AC component 574 represents the pulsatile components of optical absorption originating from the pulsatile arterial blood and the DC component 575 represents the non-pulsatile component containing contributions from non-pulsatile arterial blood, venous blood, and other tissues.

Fig. 8B shows in the middle row three diagrams illustrating the detected reflected light of a radial PPG setup. For this purpose, the axes of ordinate 581 illustrate the reflected detected light and the axes of abscissae 582 illustrate the measurement time. It becomes apparent from the three curves 583, 584, 585 that the longer the light has travelled through skin 12 of the subject (and the larger the radial distance 15 is, cf. Fig. 7B), the smaller the DC component of the reflected detected light. This is schematically illustrated by the offset of the curves 583, 584, 585, respectively. The reason for this is that more light or electromagnetic radiation 90 is absorbed by the skin region 12 if the light travels through skin by a longer path.

This dependency is also illustrated in the lowermost diagram at the bottom of Fig. 8B. This diagram illustrates the AC or DC component of the reflected detected light on the axis of ordinate 591 and the radial distance 15 on the axis of abscissae 592. The first curve 593 illustrates the DC component of the reflected detected light and the second curve 594 illustrates the ratio AC/DC of the reflected detected light. The already discussed trend becomes apparent. Further, it becomes clear that the larger the radial distance 15, the larger the ratio AC/DC (the larger the relative pulsatile component).

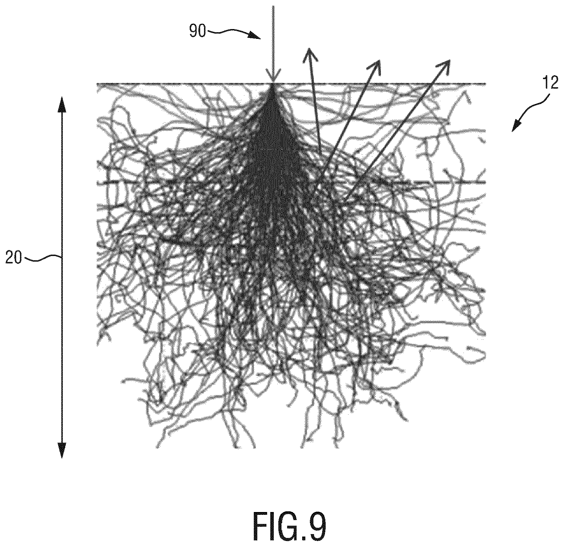

Fig. 9 shows a schematic diagram of Monte Carlo simulations of the path of light through the skin 12 of a subject for radial PPG. The light distribution inside the skin 12 is visualized to illustrate the different penetration depths 20.

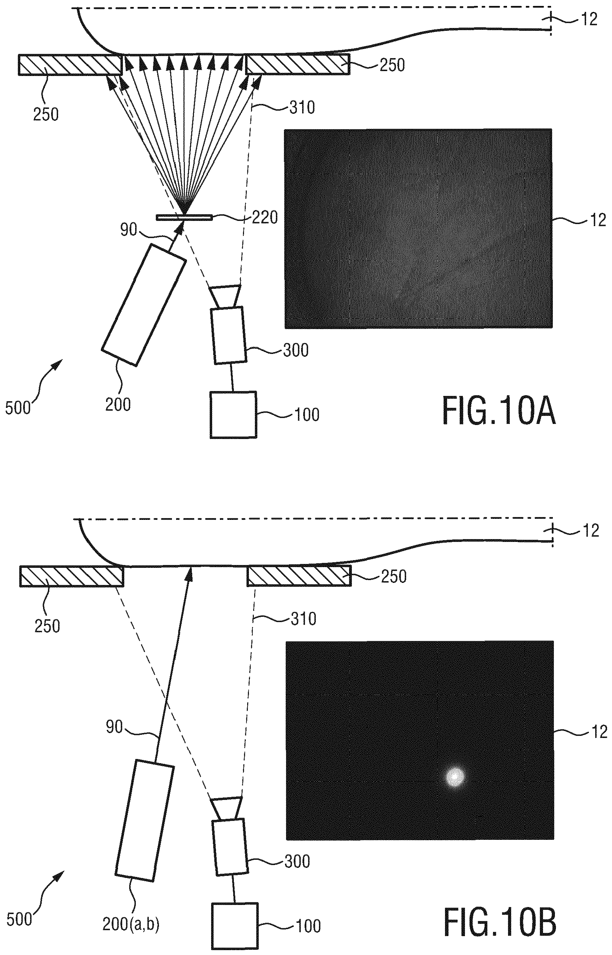

Figs. 10A and 10B show a system 500 for determining a SpO2 value of a subject according to the present invention. Said system 500 is configured to overcome the problems discussed above by combining the results of widefield PPG measurements (cf. Fig. 10A) and radial PPG measurements (cf. Fig. 10B).

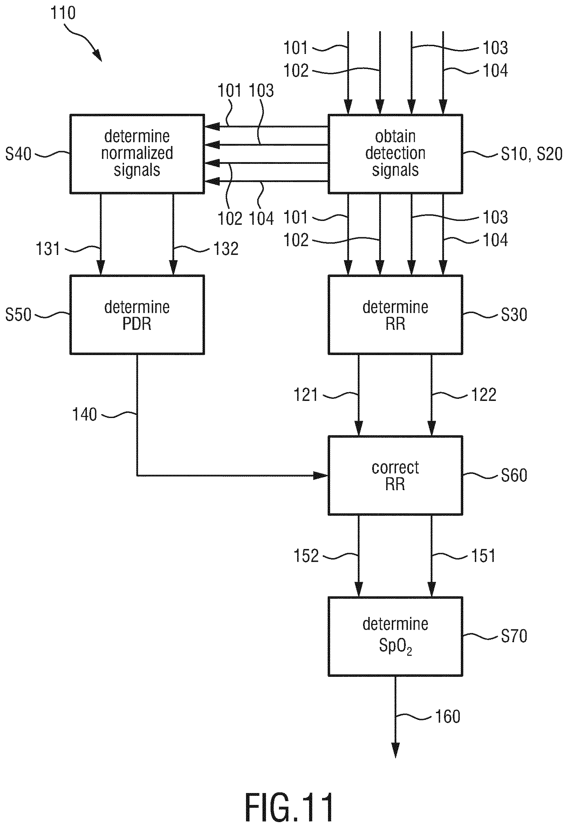

As shown in Fig. 10A, the system 500 at least comprises an illumination unit 200, a detection unit 300 and a device 100 for determining the SpO2 of a subject. A more detailed explanation of said device 100 will be given below with reference to subsequent Fig. 11.

The illumination unit 200 is configured to emit electromagnetic radiation 90 to illuminate a skin 12 of a subject. Preferably, said illumination unit 200 is configured to emit a controllable narrow beam of electromagnetic radiation 90. The electromagnetic radiation 90 is preferably located in the visible and infrared spectral range. Thus, the illumination unit 200 may be configured to emit electromagnetic radiation 90 at at least two different wavelengths and/or to alternately emit red and infrared light as electromagnetic radiation 90.

According to the embodiment shown in Fig. 10A, the system 500 may further comprise a support 250 for limiting the skin region 12 of the subject to a limited skin area to be measured. This support 250 may be placed on the skin of the subject as shown in Fig. 10A and is preferably made of a material that is non-transparent for the incoming electromagnetic radiation 90. Preferably, the skin region 12 used for measurement is thus limited to an area which comprises a skin region 12 with a homogenous surface along the area to be measured.

Additionally, the system 500 may further comprise a diffuser 220. Said diffuser 220 is configured to diffuse the controllable narrow beam of electromagnetic radiation 90 emitted by the illumination unit 200 to generate a homogenous illumination profile and/or structured light on the skin region 12 of the subject.

The detection unit 300 is preferably a camera configured to detect electromagnetic radiation 90 in the visible and infrared spectral range. The camera 300 is located such that the field of view 310 covers the area of skin 12 illuminated by the illumination unit 200.

It should be noted that widefield PPG can not only be measured, if the skin 12 of the subject is illuminated homogenously. It also works as well if the skin 12 is illuminated by structured light, such as by a spot pattern (dots, circles, stripes, etc.). In that case, the PPG signal is derived by spatial integral of all the electromagnetic radiation 90 transmitted through or reflected from the skin 12 of the subject. The processing unit 100 may be configured to perform said spatial integral.

Further, it should be noted that the generation of a homogenous illumination profile on the skin region 12 of the subject cannot only be obtained by using a illumination unit 200 configured to emit a controllable narrow beam (such as a laser) and a diffuser 220 to diffuse said narrow beam, but also by using one or even more illumination units that directly emit a homogenous illumination profile.

Fig. 10B shows an illustration of the discussed system 500 according to the present invention for the radial PPG mode. In difference to the embodiment of the system 500 shown in Fig. 10A, the system 500 shown in Fig. 10B does not comprise a diffuser 220. Hence, the controllable narrow beam of electromagnetic radiation 90 emitted by the illumination unit 220 is directly adjusted towards the skin 12 of the subject without being diffused. For this purpose, the diffuser 220 may be configured such that it may be optionally placed into the path of the emitted electromagnetic radiation 90 of the illumination unit 200 to switch between the radial PPG mode and the widefield PPG mode.

The inlets in Figs. 10A and 10B show pictures of the illumination profiles of widefield PPG (Fig. 10A) and radial PPG (Fig. 10B) on the skin region 12 of the subject.

It shall be understood that the embodiment shown in Figs. 10A and 10B is only exemplarily in that the system 500 only comprises one illumination unit 200 configured to emit a narrow beam of electromagnetic radiation 90. As already discussed above, the present invention is based on the idea to combine the results of widefield PPG and radial PPG. Hence, according to another aspect of the present invention, the system 500 may not only comprise one illumination unit configured to emit a narrow radiation beam, but further another illumination unit configured to emit a homogenous illumination profile and/or structured, i.e., a pattern of illumination spots.

According to said aspect, an optical diffuser 220 is not needed anymore. Hence, said system 500 may comprise a first illumination 200a and a second illumination unit 200b, wherein the illumination units 200a,b are by its own configured to generate the respective illumination profiles (i.e., a spot illumination and a homogenous illumination profile and/or a structured pattern).

Fig. 11 shows a flowchart illustrating a method to be executed by the device 100 for determining the SpO2 value of a subject according to the present invention.

The device 100 comprises a processing unit 110, which in a first step S10 obtains a first and second detection signal 101, 102 derived from detected electromagnetic radiation 90 at different wavelengths transmitted through or reflected from skin region 12 of the subject illuminated by spot illumination. These detection signals 101, 102 may be derived from the radial PPG setup as shown in Fig. 10B.

Further, the processing unit 110 is configured to obtain in a next step S20 a third and fourth detection signal 103, 104 derived from detected electromagnetic radiation 90 at said different wavelengths transmitted through or reflected from said skin region 12 of the subject illuminated by homogenous illumination and/or structured light, wherein the third detection signal 103 is derived from detected electromagnetic radiation at the same wavelength as the first detection signal 101 and the fourth detection signal 104 is derived from detected electromagnetic radiation at the same wavelength as the second detection signal 102. The detection signals 103, 104 may be derived from the widefield PPG setup as shown in Fig. 10B, wherein the third and first detection signal 101, 103 may be derived from electromagnetic radiation in the infrared spectral range while the second and fourth detection signals 102, 104 may be derived from electromagnetic radiation in the red spectral range.

In a next step S30, the processing unit 110 is configured to determine a first ratio of ratios (RR1) 121 from the first and second detection signal 101, 102 and a second ratio of ratios (RR2) 122 from the third and fourth detection signal 103, 104. The determination of said ratio of ratios 121, 122 is done how it has been already explained above in equation (1).

In a next step S40, the processing unit 110 determines a first normalized signal 131 by calculating the ratio of the first detection signal 101 to the third detection signal 103 and a second normalized signal 132 by calculating the ratio of the second detection signal 102 to the fourth detection signal 104. These normalized signals 131, 132 are a measure for the penetration depth 20 of the respective wavelengths (of electromagnetic radiation in the infrared spectral range and electromagnetic radiation in the red spectral range).

Then, the processing unit 110 determines in another step S50 a penetration depth ratio (PDR) 140 by calculating the ratio of the first normalized signal 131 to the second normalized signal 132. Said PDR reflects the discrepancy of the penetrations depths of electromagnetic radiation in the red spectral range and electromagnetic radiation in the infrared spectral range. This PDR is typically not only a value, but a curve PDR(r), where r is the radial distance 15 between the spot on the skin from which the radial PPG signal is measured and the illumination spot on the skin (cf. radial distance 15 in Fig. 7B). It will become apparent from the description with reference to the following figures that the curve PDR(r) is rather flat and thus may be assumed to be a value.

In a next step S60, the processing unit 110 corrects the RR1 121 and the RR2 122 by using the PDR 140 to compensate for the discrepancy in penetration depth 20 between said different wavelengths.

At a final step S70, the processing unit determines the SpO2 160 from the corrected RR1 151 and/or the corrected RR2 152. The determination of the SpO2 160 from the corrected ratio of ratios is done how it has been already explained with reference to Fig. 1.

The steps S10-S70 executed by the processing unit 110 of the device 100 are explained in detail with reference to the following figures.

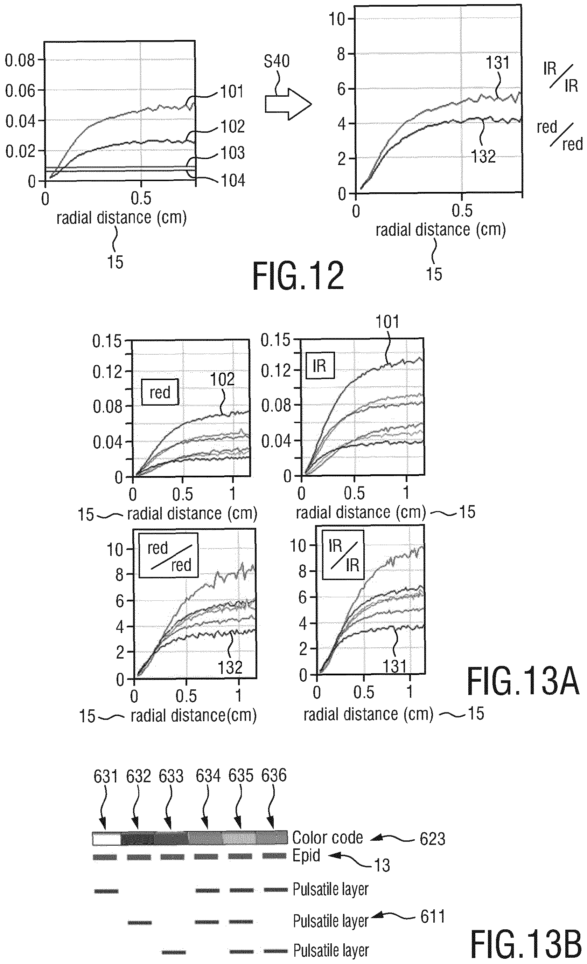

Fig. 12 shows schematic diagrams illustrating the first processing step of the detection signals obtained by the device 100 by illustrating the step of determining S40 a first normalized signal 131 and a second normalized signal 132. The diagram on the left of Fig. 12 illustrates the detection signals 101, 102, 103, 104 as functions of the radial distance 15 (cf. Fig. 7B for further explanations of the radial distance 15).

The first detection signal 101 is derived from electromagnetic radiation in the infrared spectral range transmitted through or reflected from a skin region of a subject illuminated by a spot illumination (radial PPG). The second detection signal 102 is derived from electromagnetic radiation in the red spectral range transmitted through or reflected from a skin region of a subject illuminated by a spot illumination (radial PPG). The third detection signal 103 is derived from electromagnetic radiation in the infrared range transmitted through or reflected from a skin region of a subject illuminated by homogenous illuminations and/or structured light (widefield PPG). The fourth detection signal 104 is derived from electromagnetic radiation in the infrared range transmitted through or reflected from a skin region of a subject illuminated by homogenous illuminations and/or structured light (widefield PPG).

The third and the fourth detection signal 103, 104 derived from widefield PPG are constant and independent on the radial distance 15, while the first and the second detection signals 101, 102 are functions depending on the radial distance 15.

As explained above, the processing unit 110 determines in a step S40 a first normalized signal 131 by calculating the ratio of the first detection signal 101 to the third detection signal 103 and a second normalized signal 132 by calculating the ratio of the second detection signal 102 to the fourth detection signal 104. Hence, Fig. 12 illustrates in the right diagram the first normalized signal 131 which is derived from electromagnetic radiation in the infrared spectral range and the second detection signal 132 which is derived from electromagnetic radiation in the red spectral range versus the radial distance 15, respectively.

The diagrams shown in Fig. 12 and the diagrams shown in the subsequent Figs. 13-23 are preferably all visualized on a monitor which is connected to the device 100 for determining SpO2 of a subject.

Figs. 13A and 13B show schematic diagrams illustrating the influence of different skin layers 631-636 on the measured signals. The skin layers 631-636 with one, two or three pulsatile layers 611 and one layer of epidermis 13 are schematically illustrated in Fig. 13B. The six different skin layers 631-636 are further indicated by six different color codes 623 with different colors or grey shades. These grey shades are also used for the corresponding curves in Fig. 13A.

Fig. 13A shows in the first row the radial PPG curves (radPPG(r)) for the six different skin geometries. If appropriate, the radial distance 15 will be denoted by 'r' in the following. The unit of the radial distance 15 in Fig. 13A is centimeter.

The left diagram in the first row of Fig. 13A shows the second detection signal 102 and the right diagram shows the first detection signal 101 for the six different skin layers 631-636. It becomes apparent from the left diagram that the curves do not have a clear relationship with the PPG skin-geometry. The highest curve belongs to the fifth skin layer 635 as this skin layer is modelled for three pulsatile layers 611 instead of one or two. Thus, this curve has the largest intensity.

It can also be seen that the curves are quite strongly dependent on the optical depth of the PPG source. The curves show much larger values for the skin geometry relating to the third skin layer 633 than the skin geometry relating to the first skin layer 631. Further, the curves belonging to electromagnetic radiation in the infrared range show slightly larger values than the curves belonging to electromagnetic radiation in the red spectral range. This can be explained by the fact that infrared light penetrates slightly less deep into the skin than red light. The pulsatile layers 611 are thus optically deeper for infrared than for red light.

The values for infrared shown in the first row in the right diagram are roughly twice as large compared to those for red, reflecting the larger absorption coefficient of the pulsatile blood in the infrared spectral range.

The left diagram in the second row of Fig. 13A shows the second normalized signal 132 and the right diagram shows the first normalized signal 131 for the six different skin layers 631-636. These curves illustrate that the values are larger when the physical depth of pulsatile layers 611 is larger. This can be seen by comparing the highest curve, which belongs to the third skin layer 633, to the lowest curve, which belongs to the first skin layer 631.

An Optical Depth Index (ODI) may be defined by taking the value of the first normalized signal 131 and the value of the second normalized signal 132 for r = 1 cm. This ODI is not a true physical depth, rather an expression of the relative optical depth of the source of the PPG: one or more pulsatile layer(s) (cf. Fig. 13B). The word 'relative' means that the ODI can be used for comparing it for different wavelengths, but the ODIs can also be used to compare the relative optical depths for different anatomical locations.

The larger ODI value for infrared compared to red (cf. the values of the first normalized signal 131 to the values of the second normalized signal 132 at r = 1 cm in Fig. 13A) obviously result from the different penetration depths for these wavelengths. The actual physical depth of the pulsatile layer is the same for both wavelengths. It only 'appears' deeper (larger ODI) for infrared, because infrared has a smaller penetration depth. The penetration depth is inversely proportional to the ODI.

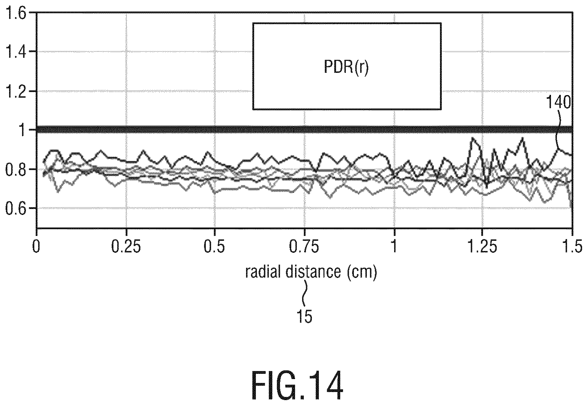

As explained above with reference to Fig. 11, the processing unit 110 is further configured to determine S50 a penetration depth ratio (PDR) 140 by calculating the ratio of the first normalized signal 131 to the second normalized signal 132. Said PDR 140 reflects the discrepancy of the penetrations depths of electromagnetic radiation in the red spectral range and electromagnetic radiation in the infrared spectral range. With the knowledge that the normalized signals 131, 132 are a measure for the penetration depth of a wavelength, these normalized signals 131, 132 are thus used to obtain a measure for the relative penetration depths: PDR 140. This is illustrated in the following Fig. 14.

Fig. 14 shows said PDR 140 in dependence on the radial distance 15. The PDR 140 is again calculated for the six different skin layers 631-636. It becomes apparent that the PDR(r) 140 is always smaller than 1, indicating that red 'sees' deeper than infrared. Since the discrepancy in penetration depths between red and infrared radiation is thought to be a cause of the inaccuracy of SpO2 (cf. explanations with reference to Figs. 6A and 6B above) of state of the art pulse oximetry, it is used here for obtaining more accurate SpO2 values, which will be explained in more detail in the following.

As shown in Fig. 14, the PDR(r) 140 is a curve: it is a function of radial distance 15 (between the illumination spot on the skin region 12 and the spot from which the PPG signal is measured; cf. explanations with reference to Fig. 7B for more details). However, it is seen in Fig. 14 that for all skin geometries the curve PDR(r) 140 is rather flat. Thus, it is assumed in the following that the PDR 140 is a constant value. This can be either done by just taking the median value of PDR(r) 140, or simply by using the ratio of the ODI values: (PDI=ODI($\lambda$1)/ODI($\lambda$2)).

As the processing unit 110 is preferably connected to a monitor (not shown) which may visualize the diagrams shown in Figs. 12-23, the processing unit 110 may be configured to generate a warning signal to visualize on the monitor if the PDR 140 is not within a predetermined range. This would indicate to the user that the used wavelengths do not appear to probe the same depths and associated pulsatile vasculature. Further, it indicates that the normal, uncorrected SpO2 which bases on the uncorrected ratio of ratios 121, 122, is likely to not give an accurate SpO2.

As explained with reference to Fig. 11, the processing unit 110 further determines in a step S30 a first ratio of ratios (RR1) 121 from the first and second detection signals 101, 102 and a second ratio of ratios (RR2) 122 from the third and fourth detection signals 103, 104. The determination of said ratio of ratios 121, 122 is done how it has been already explained above in equation (2).

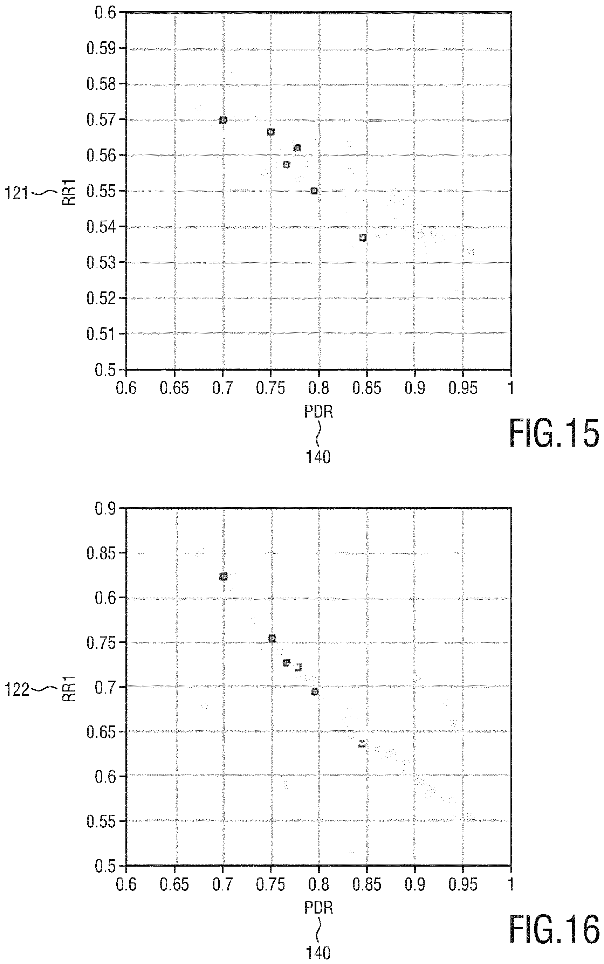

Fig. 15 shows a diagram illustrating the RR1 121 versus PDR 140. Fig. 16 shows a diagram illustrating the RR2 122 versus PDR 140. The PDR 140 is extracted for all six different skin layers 631-636 by taking a constant value for the PDR(r) curves, respectively (cf. Fig. 14).

It can be seen in Figs. 15 and 16 that the variation for radial PPG (RR1=0.54-0.57) is much smaller than the variation in widefield PPG (RR2=0.63-0.82). It is further seen that both, RR1 and RR2, have a somewhat linear relationship with PDR 140, showing a promising opportunity to use the PDR 140.

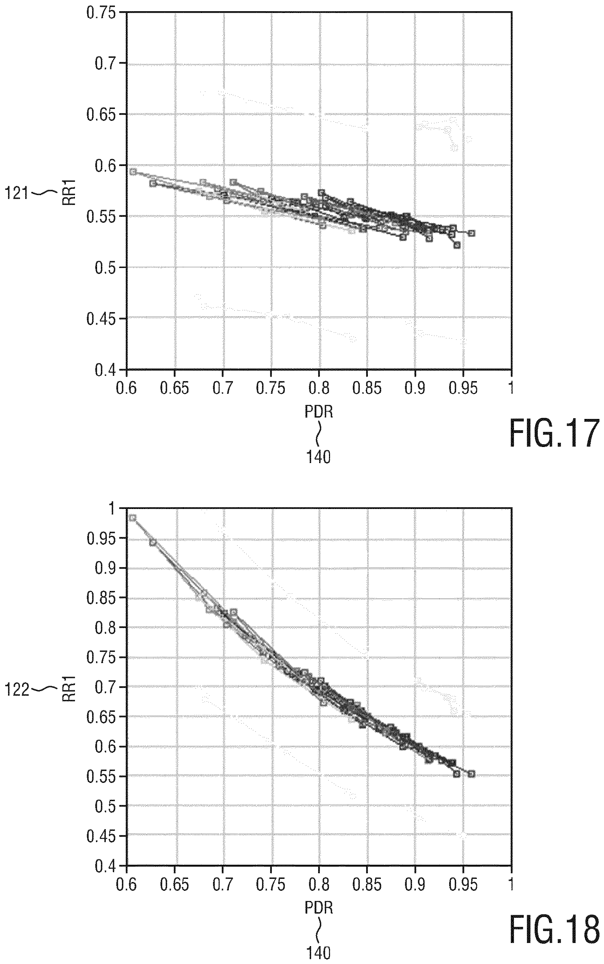

Figs. 17 and 18 show further diagrams illustrating the RR1 121 and the RR2 122 versus PDR 140. Contrary to the data points shown in Figs. 15 and 16, Figs. 17 and 18 show even more results for a large range of skin properties, simulating different individuals and also different physiological states. Different degrees of venous oxygenation (e.g., 0.02 to 0.08 in the non-pulsatile layer) and various combinations of the scattering coefficients (150 and 250 $cm^{-1}$) and anisotropy factor (0.7 and 0.74) have been used and tested. It can be seen by comparing Figs. 17 and 18 that the RR1 (radial RR) 121 vs. PDR 140 shows a much smaller correlation with the PDR 140 than the RR2 (widefield RR) 122 vs. PDR 140. This is due to the much smaller variation of RR1 (cf. also Figs. 15 and 16). This means that SpO2 160 derived from RR1 is likely to be more accurate than from RR2, which shows a huge variation in Figs. 16 and 18.

In the following, it is described how to use the relationships between RR1 121 and/or RR2 122 and PDR 140 shown in the last figures to arrive at corrected RR1 151 and/or corrected RR2 152 that allow determining SpO2 160 of a subject with higher accuracy.

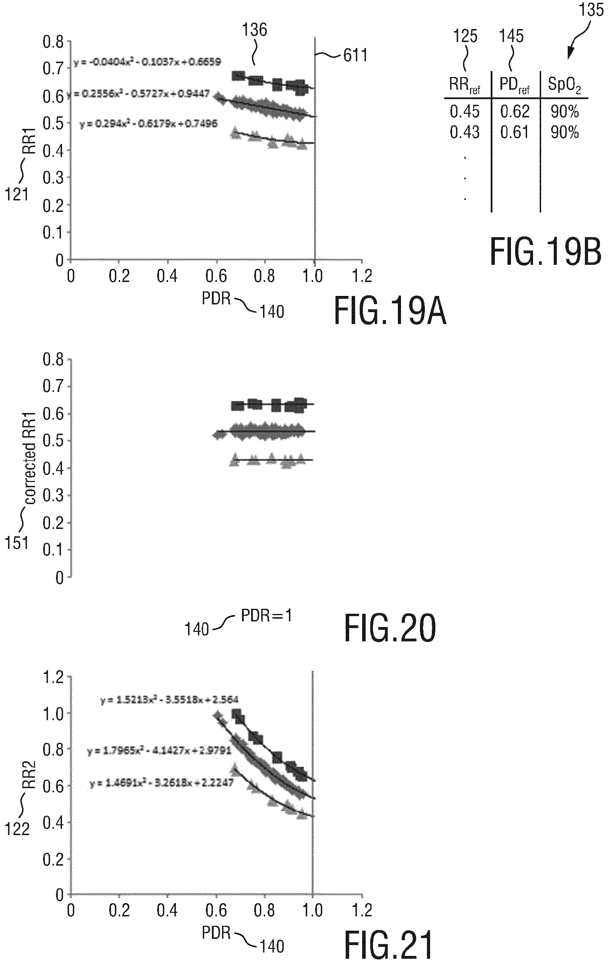

Fig. 19 shows a diagram (Fig. 19A) and a lookup table 135 (Fig. 19B) illustrating an example of correction of RR1. The RR1 121 is illustrated versus PDR 140. Three different calibration curves 136 describing the relationship between reference ratio of ratios ($RR_{ref}$) 125 and reference penetration depths ($PDR_{ref}$) 145 are shown for different SpO2 and different RR values. These calibration curves 136 may be based on $RR_{ref}$ 125 and $PDR_{ref}$ 145 stored in a lookup table 135 as shown in Fig. 19B. The curves shown in Fig. 19A are generated with Monte Carlo simulations using values for skin constituent concentrations and optical properties in realistic ranges.

The processing unit 110 is configured to select a matching calibration curve 136 to correct the RR1 121 by extrapolating said matching curve to the $PDR_{ref}$ equal to 1 and setting the RR1 121 to the corresponding $RR_{ref}$ 125. Thus, the corrected RR1 is obtained by extracting the RR1 value at the crossing point of the matching calibration curve 136 with the vertical line 611 illustrated in Fig. 19A. The matching calibration curve 136 is preferably selected by selecting the curve, which is closest to the data point obtained in Fig. 19A if the determined RR1 121 and the determined PDR 140 were visualized in the diagram.

Fig. 20 shows a corresponding diagram showing the constant corrected RR1 151. This constant corrected RR1 151 is obtained by the procedure as explained with reference to Figs. 19A above.

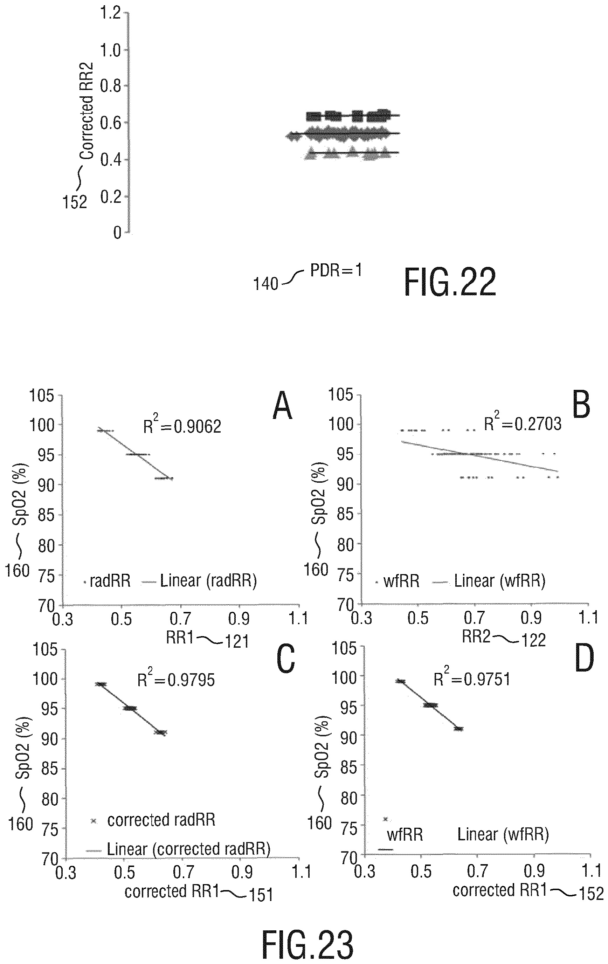

Figs. 21 and 22 show the same correction procedure for RR2 122 to obtain a corrected RR2 152. Preferably, said correction procedure is also done by comparing the RR2 122 to a lookup table 135 of $RR_{ref}$ 125 and $PDR_{ref}$ 145 (not shown) and by the same extrapolation procedure, which has been explained with reference to Figs. 19A and 20 for RR1.

Figs. 23A-D show diagrams illustrating the determined SpO2 versus RR. Figs. 23A and 23B show diagrams illustrating the determined SpO2 160 in dependence on RR1 121 and RR2 122. Figs. 23C and 23D show diagrams illustrating the determined SpO2 160 in dependence on the corrected RR1 151 and corrected RR2 152.

It can be seen that the corrected RR1 151 and corrected RR2 show much smaller spread compared to the (uncorrected) RR1 121 and RR2 122. This results in much tighter SpO2 calibration curves. This implies that using corrected RR1 151 and corrected RR2 152 allows obtaining a more accurate SpO2, in particular compared to RR2 (widefield RR), which is relatively inaccurate and typically used for various stand pulse oximeters.

The relationships between the various $RR_{ref}$ 125 and SpO2 160 may be determined by models, numerical simulations, but also by empirical measurements on a large number of individuals, different anatomical locations and various actual SpO2 levels.

Similar to normal pulse oximetry procedures, a series of widefield RR (RR2), radial RR (RR1) and SpO2 from blood gas analysis may be acquired to determine the relationships. A calibration procedure to do so provides reference data $RR_{ref}$ 125 and $PDR_{ref}$ 145.

Just like in current calibration procedures, multiple volunteers may be asked to breath a mixture with varying O2 concentrations which results in lower SpO2 values (e.g., 70-95%) than normal (95-100%). Rather than just take one measurement with a pulse oximeter, both widefield PPG and radial PPG are measured with red and infrared electromagnetic radiation 90. For each measurement the corresponding PDR 140 is calculated.

Once such a calibration curve 136 or calibration lookup table 135 is created, a pulse oximeter device 100 could either be measuring in continuous radial PPG mode, with a widefield PPG measurement every now and then to update the PDR mode. Alternatively, the pulse oximeter device 100 could also measure continuous widefield PPG with every now and then a radial PPG measurement.

Either way, the eventual SpO2 output will be based on the corrected RR1 151 and/or the corrected RR2, rather than RR1 121 and/or RR2 resulting in more accurate SpO2 values of a subject.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. Device (100) for determining oxygen saturation, SpO2, (160) of a subject, said device (100) comprising a processing unit (110) configured to:

   - obtain (S10) a first and second detection signal (101, 102) derived from detected electromagnetic radiation (90) at different wavelengths transmitted through or reflected from a skin region (12) of the subject illuminated by spot illumination;
   - obtain (S20) a third and fourth detection signal (103, 104) derived from detected electromagnetic radiation (90) at said different wavelengths transmitted through or reflected from said skin region (12) of the subject illuminated by homogenous illumination and/or by a spot pattern of illumination, wherein the third detection signal (103) is derived from detected electromagnetic radiation at the same wavelength as the first detection signal (101) and the fourth detection signal (104) is derived from detected electromagnetic radiation at the same wavelength as the second detection signal (102);
   - determine (S30) a first ratio of ratios, RR1, (121) from the first and second detection signals (101, 102) and a second ratio of ratios, RR2, (122) from the third and fourth detection signals (103, 104);
   - determine (S40) a first normalized signal (131) by calculating the ratio of the first detection signal (101) to the third detection signal (103) and a second normalized signal (132) by calculating the ratio of the second detection signal (102) to the fourth detection signal (104);
   - determine (S50) a penetration depth ratio, PDR, (140) by calculating the ratio of the first normalized signal (131) to the second normalized signal (132);
   - correct (S60) the RR1 (121) and the RR2 (122) using the PDR (140) to compensate for the discrepancy in penetration depth (20) between said different wavelengths; and
   - determine (S70) the SpO2 (160) from the corrected RR1(151) and/or the corrected RR2 (152).

2. Device (100) according to claim 1, wherein the processing unit (110) is configured to derive, in case of a spot pattern of illumination, the third and fourth detection signals (103, 104) by a spatial integral of all the electromagnetic radiation (90) transmitted through or reflected from said skin region (12).

3. Device (100) according to any one of the preceding claims, wherein the processing unit (110) is configured to correct the RR1 (121) and/or the RR2 (122) by use of reference ratio of ratios $RR_{ref}$ (125) and reference penetration depth ratios $PDR_{ref}$ (145).

4. Device (100) according to any one of the preceding claims, wherein the processing unit (110) is configured to correct the RR1 (121) and/or the RR2 (122) by comparing said RR1 (121) and/or said RR2 (122) and said PDR (140) to a lookup table (135) of reference ratio of ratios $RR_{ref}$ (125) and reference penetration depth ratio $PDR_{ref}$ (145).

5. Device (100) according to any one of the preceding claims, wherein the processing unit (110) is configured to use calibration curves (136) describing the relationship between reference ratio of ratios $RR_{ref}$ (125) and reference penetration depth ratios $PDR_{ref}$(145) for different SpO2 values (160) to compare the PDR (140) and the RR1 (121) and/or RR2 (122) to said calibration curves (136).

6. Device (100) according to claim 5, wherein the processing unit (110) is configured to select a matching calibration curve to correct the RR1 (121) and/or the RR2 (122) by extrapolating said matching curve to the $PDR_{ref}$(145) equal to 1 and setting the RR1 (121) and/or the RR2 (122) to the corresponding $RR_{ref}$ (125).

7. Device according to claim 5 or 6, wherein the processing unit (110) is configured to use different calibration curves (136) to correct the RR1 (121) and/or the RR2 (122).

**8.** A system (500) for determining oxygen saturation, SpO2, (160) of a subject, said system (500) comprising:

an illumination unit (200) configured to emit a narrow radiation beam of electromagnetic radiation to illuminate a skin region (12) of the subject by a spot illumination;
an optical diffuser (220) that can selectively be arranged within or outside of the path of the emitted light of the illumination unit (200), wherein the optical diffuser (220) is configured to diffuse the electromagnetic radiation (90) emitted by the illumination unit (200) to illuminate the skin region (12) of the subject homogenously and/or by a spot pattern;
a detection unit (300) configured to detect the electromagnetic radiation (90) transmitted through or reflected from the skin region (12) of the subject and to derive detection signals (101, 102, 103, 104) from the detected electromagnetic radiation (90); and
a device (100) for determining the SpO2 (160) of the subject according to any one of the preceding claims based on the detection signals (101, 102, 103, 104).

**9.** System (500) according to claim 8, wherein the illumination unit (200) is configured to emit electromagnetic radiation (90) at at least two different wavelengths and/or to alternately emit red light and infrared light as electromagnetic radiation (90).

**10.** System according to any one of claims 8 or 9, wherein the illumination unit (200) and the detection unit (300) are either both in direct physical contact to the skin (12) of the subject or not in direct physical contact to the skin (12) of the subject.

**11.** A system (500) for determining an oxygen saturation, SpO2, (160) of a subject, said system (500) comprising:

a first illumination unit (200a) configured to emit a narrow radiation beam of electromagnetic radiation to illuminate a skin region (12) of the subject by a spot illumination;
a second illumination unit (200b) configured to emit a homogenous illumination profile of electromagnetic radiation and/or a spot pattern to illuminate the skin region (12) of the subject homogenously and/or by a spot pattern;
a detection unit (300) configured to detect the electromagnetic radiation (90) transmitted through or reflected from the skin region (12) of the subject and to derive detection signals (101, 102, 103, 104) from the detected electromagnetic radiation (90); and
a device (100) for determining the SpO2 (160) of the subject according to any one of the preceding claims based on the detection signals (101, 102, 103, 104).

**12.** System according to any one of claims 8 to 11, wherein the detection unit (300) is an optical sensor and comprises a plurality of detection elements, in particular an array of photo diodes, a CCD array or a CMOS array.

**13.** A method for determining oxygen saturation, SpO2, (160) of a subject, said method comprising the steps of:

- obtaining (S10) a first and second detection signal (101, 102) derived from detected electromagnetic radiation (90) at different wavelengths transmitted through or reflected from a skin region (12) of the subject illuminated by spot illumination;
- obtaining (S20) a third and fourth detection signal (103, 104) derived from detected electromagnetic radiation (90) at said different wavelengths transmitted through or reflected from said skin region (12) of the subject illuminated by homogenous and/or spot illumination, wherein the third detection signal (103) is derived from detected electromagnetic radiation at the same wavelength as the first detection signal (101) and the fourth detection signal (104) is derived from detected electromagnetic radiation at the same wavelength as the second detection signal (102);
- determining (S30) a first ratio of ratios, RR1, (121) from the first and second detection signals (101, 102) and a second ratio of ratios, RR2, (122) from the third and fourth detection signals (103, 104);
- determining (S40) a first normalized signal (131) by calculating the ratio of the first detection signal (101) to the third detection signal (103) and a second normalized signal (132) by calculating the ratio of the second detection signal (102) to the fourth detection signal (104);
- determining (S50) a penetration depth ratio, PDR, (140) by calculating the ratio of the first normalized signal (131) to the second normalized signal (132);
- correcting (S60) the RR1 (121) and the RR2 (122) using the PDR (140) to compensate for the discrepancy in penetration depth (20) between said different wavelengths; and
- determining (S70) the SpO2 (160) from the corrected RR1 (151) and/or the corrected R2 (152).

**14.** Computer program comprising program code means for causing a computer to carry out the steps of the method as claimed in claim 13 when said computer program is carried out on the computer.

FIG.1

$$y = 118 - 45.9\,x$$

FIG.2

FIG.3A

FIG.3B

FIG.4A

FIG.4B

FIG.5A

FIG.5B

FIG.6A

FIG.6B

widefield PPG

**FIG.7A**

radial PPG

**FIG.7B**

widefield PPG

**FIG.8A**

radial PPG

**FIG.8B**

FIG.9

FIG.10A

FIG.10B

FIG.11

FIG.12

FIG.13A

FIG.13B

FIG.14

FIG.15

FIG.16

FIG.17

FIG.18

$y = -0.0404x^2 - 0.1037x + 0.6659$

$y = 0.2556x^2 - 0.5727x + 0.9447$

$y = 0.294x^2 - 0.6179x + 0.7496$

FIG.19A

| RR_ref | PD_ref | SpO_2 |
|--------|--------|-------|
| 0.45 | 0.62 | 90% |
| 0.43 | 0.61 | 90% |
| . | | |
| . | | |
| . | | |

FIG.19B

FIG.20

PDR=1

$y = 1.5213x^2 - 3.5518x + 2.564$

$y = 1.7965x^2 - 4.1427x + 2.9791$

$y = 1.4691x^2 - 3.2618x + 2.2247$

FIG.21

FIG.22

FIG.23

Europäisches
Patentamt

European
Patent Office

Office européen
des brevets

# EUROPEAN SEARCH REPORT

Application Number

EP 19 21 7847

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/188919 A1 (AL-ALI AMMAR [US] ET AL) 6 July 2017 (2017-07-06) * figures 1A, 2 * * paragraphs [0039], [0043], [0047] - [0049] * | 1-7,13, 14 | INV. A61B5/1455 A61B5/00 |
| A | US 2019/167124 A1 (VERKRUIJSSE WILLEM [NL] ET AL) 6 June 2019 (2019-06-06) * paragraphs [0099] - [0100], [0145] - [0149]; figures 15A-C * | 1-14 | |
| A | US 2019/286233 A1 (NEWBERRY ROBERT [US]) 19 September 2019 (2019-09-19) * paragraphs [0132] - [0135] * * figure 2 * | 1-14 | |
| A | US 2013/006074 A1 (POLOGE JONAS A [US]) 3 January 2013 (2013-01-03) * paragraphs [0017], [0024] - [0027] * | 1-14 | |

TECHNICAL FIELDS SEARCHED (IPC)

A61B

The present search report has been drawn up for all claims

| Place of search | Date of completion of the search | Examiner |
|---|---|---|
| The Hague | 4 May 2020 | Trattner, Barbara |

EPO FORM 1503 03.82 (P04C01)

EP 3 838 144 A1

## ANNEX TO THE EUROPEAN SEARCH REPORT
## ON EUROPEAN PATENT APPLICATION NO.

EP 19 21 7847

This annex lists the patent family members relating to the patent documents cited in the above-mentioned European search report.
The members are as contained in the European Patent Office EDP file on
The European Patent Office is in no way liable for these particulars which are merely given for the purpose of information.

04-05-2020

| Patent document cited in search report | | Publication date | Patent family member(s) | | Publication date |
|---|---|---|---|---|---|
| US 2017188919 | A1 | 06-07-2017 | AT | 521277 T | 15-09-2011 |
| | | | AU | 4214199 A | 20-12-1999 |
| | | | CA | 2333062 A1 | 09-12-1999 |
| | | | EP | 1082050 A1 | 14-03-2001 |
| | | | EP | 2319398 A1 | 11-05-2011 |
| | | | JP | 2002516689 A | 11-06-2002 |
| | | | US | 6334065 B1 | 25-12-2001 |
| | | | US | 2002082488 A1 | 27-06-2002 |
| | | | US | 2004059209 A1 | 25-03-2004 |
| | | | US | 2005197551 A1 | 08-09-2005 |
| | | | US | 2006258923 A1 | 16-11-2006 |
| | | | US | 2006258924 A1 | 16-11-2006 |
| | | | US | 2006258925 A1 | 16-11-2006 |
| | | | US | 2006270920 A1 | 30-11-2006 |
| | | | US | 2006281983 A1 | 14-12-2006 |
| | | | US | 2013197330 A1 | 01-08-2013 |
| | | | US | 2015018650 A1 | 15-01-2015 |
| | | | US | 2017188919 A1 | 06-07-2017 |
| | | | WO | 9962399 A1 | 09-12-1999 |
| US 2019167124 | A1 | 06-06-2019 | US | 2019167124 A1 | 06-06-2019 |
| | | | WO | 2018029123 A1 | 15-02-2018 |
| US 2019286233 | A1 | 19-09-2019 | US | 2019286233 A1 | 19-09-2019 |
| | | | US | 2020004336 A1 | 02-01-2020 |
| | | | WO | 2019177700 A2 | 19-09-2019 |
| US 2013006074 | A1 | 03-01-2013 | EP | 2725979 A2 | 07-05-2014 |
| | | | US | 2013006074 A1 | 03-01-2013 |
| | | | WO | 2013003077 A2 | 03-01-2013 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **A. HELLSTROM et al.** Retinopathy of prematurity. *The Lancet,* 2013, vol. 382 (9902

- **O.D. SAUGSTAD ; D. AUNE.** Optimal Oxygenation of Extremely Low Birth Weight Infants: A Meta-Analysis and Systematic Review of the Oxygen Saturation Target Studies. *Neonatology,* vol. 105, 2014